# EUROPÄISCHE PATENTSCHRIFT

(11) **EP 3 042 856 B1**
(45) Veröffentlichungstag und Bekanntmachung des Hinweises auf die Patenterteilung: **01.02.2017**
(21) Anmeldenummer: 15003046.8
(22) Anmeldetag: 24.10.2015
(51) Int. Cl.: B65B 55/02, B65B 3/00, B65B 7/28, A61K 9/19

(54) **VORRICHTUNG ZUM PHARMAZEUTISCHEN VERSCHLIESSEN VON MIT EINEM HOCHREINEN PRODUKT GEFÜLLTEN VIALS IM ANSCHLUSS AN EINEN GEFRIERTROCKNUNGSPROZESS, INSBESONDERE UNTER REINRAUMBEDINGUNGEN**
DEVICE FOR PHARMACEUTICAL SEALING OF VIALS FILLED WITH A HIGH PURITY PRODUCT FOLLOWING A FREEZE-DRYING PROCESS, IN PARTICULAR IN CLEAN ROOM CONDITIONS
DISPOSITIF DE FERMETURE PHARMACEUTIQUE DE FLACONS REMPLIS D'UN PRODUIT EXTREMEMENT PUR A LA SUITE D'UN PROCESSUS DE LYOPHILISATION, EN PARTICULIER DANS DES CONDITIONS DE SALLE BLANCHE

(30) Priorität: 09.01.2015 DE 102015000318
(43) Veröffentlichungstag der Anmeldung: 13.07.2016
(73) Patentinhaber: HOF Sonderanlagenbau GmbH, 35102 Lohra (DE)
(72) Erfinder: HOF, Hans-Georg, 35075 Gladenbach (DE)
(74) Vertreter: Patentanwälte Walther Hinz Bayer PartGmbB

(56) Entgegenhaltungen:
- EP-A1- 0 849 173
- WO-A1-95/34078
- WO-A1-2011/015453
- US-A1- 2014 093 335

## Beschreibung

Die vorliegende Erfindung betrifft eine Vorrichtung zum pharmazeutischen Verschließen von Vials insbesondere unter Reinraumbedingungen gemäß dem Oberbegriff des Anspruches 1.

Die Gefriertrocknung von pharmazeutischen Produkten (oder auch anderen Stoffen) erfolgt in Vials, welche offen in eine Gefriertrocknungsanlage gebracht werden. Dabei werden in dem Beladebereich vor der Gefriertrocknungsanlage, in dem sich die offenen Vials bewegen, Reinraumbedingungen der Klasse A angestrebt, um eine Kontamination der in den Vials befindlichen Produkte zu verhindern.

Auf jedem Vial befindet sich lose in die Öffnung des Vials eingesetzt ein Stopfen, der nach Abschluß des Gefriertrocknungsvorganges in das Vial hineingedrückt wird, um das Vial zu verschließen. Dieser Stopfen verschließt zwar das Vial, stellt aber kein Verschluß im pharmazeutischen Sinne dar, denn unter bestimmten Bedingungen kann an dem Stopfen vorbei eine Kontamination des Produktes erfolgen. Um auch unter extremen Bedingungen eine Kontamination des Produktes im Vial zu verhindern wird zusätzlich zum Stopfen auf das Vial noch ein pharmazeutisch sicherer Verschluss angebracht. Dies ist häufig eine metallische Kappe, die über den Stopfen am Vial gestülpt wird und dicht anliegend aufgebördelt wird.

Um die noch offenen oder, wie oben beschrieben, teilweise verschlossenen Vials von der Gefriertrocknungsanlage zu einer Verschließvorrichtung zu transportieren ist in der WO 2011/015453 A1 eine Vorrichtung zum Herausholen der Vials aus einer Gefriertrocknungsanlage beschrieben, die die Vials auf einer Transportvorrichtung abstellt. Diese Transportvorrichtung kann beliebig in der Halle bewegt werden und bringt die Vials zu der Verschließvorrichtung.

Damit auch nach der Gefriertrocknung keine Kontamination des hochreinen, vorzugsweise pharmazeutischen, Produktes erfolgt, werden auch beim Entladen der Gefriertrocknungsanlage und beim Transport der Vials von der Gefriertrocknungsanlage bis zur Verschließvorrichtung, sowie beim pharmazueutischen Verschließen des Vials mit einem metallischen Verschluss, Reinraumbedingungen zumindest der Klasse A angestrebt. Aus diesem Grunde stehen die entsprechenden Gefriertrocknungsanlagen samt Entladestation, Transportvorrichtung und Verschließvorrichtung in einem großen Raum mit entsprechender Reinraumklassifizierung.

Ein Gefriertrocknungsprozeß dauert viele Stunden, je nach Produkt bis zu 60 Stunden. Das Entladen der Gefriertrocknungsanlage und das pharmazeutische Verschließen der Vials geht sehr viel schneller, selbst wenn in einer Gefriertrocknungsanlage mehrere tausend Vials gleichzeitig gefriergetrocknet werden. Aus diesem Grund ist es üblich eine einzige Verschließvorrichtung für mehrere Gefriertrocknunganlagen vorzusehen. Gleiches gilt auch für Entlade- und Transportvorrichtungen. Damit eine Entladevorrichtung und/oder Transportvorrichtung mehrere Gefriertrocknungsanlagen bedienen kann, sind diese auf Schienen vor den in Reihe angeordneten Gefriertrockungsanlagen angebracht und können zu jeder dieser Gefriertrocknungsanlagen hin gefahren werden. Über die Transportvorrichtung werden die Vials dann zu der Verschließvorrichtung transportiert. Dies alles geschieht in einem entsprechend großen Raum der erforderlichen Reinraumkategorie.

Aus POSLOVSKI, Matthias: Multibatch-Produktion von Plasma mit Isolator und Gefriertrocknung. Prozesstechnik Online, 22. Mai 2014 und aus der DE 10 2009 027 452 A1 ist eine Vorrichtung zum Füllen und Verschließen von pharmazeutischen Behältnissen bekannt, bei der innerhalb eines Gehäuses die zum Verschließen notwendigen Anlagen vorgesehen sind, wobei an der Vorrichtung eine Luftanlage zum Bereitstellen hochreiner Luft innerhalb des Gehäuses vorgesehen ist. Die zu verschließenden Behältnisse, in der Regel Vials, werden über eine entsprechende Zuführeinrichtung in die Vorrichtung zum Verschließen der Behältnisse geführt. Es versteht sich, dass der Raum, in dem die Transportvorrichtung steht, ebenfalls Reinraumbedingungen, zumindest der Klasse A, erfüllt.

Der Betrieb eines derart großen Raumes unter Reinraumbedingungen verursacht sehr hohe Investitions- und Betriebskosten, weil eine große Menge Luft hochrein gehalten werden muss.

Davon ausgehend liegt der vorliegenden Erfindung die Aufgabe zu Grunde, eine Vorrichtung zum pharmazeutischen Verschließen von Vials der eingangs genannten Art zu schaffen, mit der die Kosten zur Realisierung der Reinraumbedingungen gering gehalten werden.

Als technische Lösung dieser Aufgabe wird erfindungsgemäß eine Vorrichtung mit den Merkmalen des Anspruches 1 vorgeschlagen. Vorteilhafte Weiterbildungen dieser Vorrichtung sind den Unteransprüchen zu entnehmen.

Eine nach dieser technischen Lehre ausgebildete Vorrichtung zum Verschließen von Vials hat den Vorteil, dass mit dem Gehäuse unmittelbar an der Verschließvorrichtung, also lokal begrenzt, Reinraumbedingungen geschaffen werden, mit der Folge, dass die Investitions- und die Betriebskosten dieses kleinen Reinraumes sehr niedrig sind.

In einer bevorzugten Ausführungsform ist die Station mit all ihren Aufbauten verfahrbar ausgeführt, damit eine einzige Verschließvorrichtung mehrere Gefriertrocknungsanlagen bedienen kann, was die Investitionskosten niedrig hält. Auch wird hierdurch die Transportvorrichtung vergleichsweise klein ausgeführt, was zu einer weiteren Reduzierung der Investitions- und Betriebskosten führt.

Dabei hat es sich als vorteilhaft herausgestellt, die Station entlang einer Führungsschiene von einer ersten Gefriertrocknungsanlage zu einer zweiten Gefriertrocknungsanlage zu verfahren. Hierdurch ist eine erneute Ausrichtung der Vorrichtung nach dem Verfahren entbehrlich, denn die Station steht immer im richtigen Abstand zur Gefriertrocknungsanlage.

In einer weiteren, bevorzugten Ausführungsform ist am Gehäuse eine Luftanlage vorgesehen, mittels der hochreine Luft in das Innere des Gehäuses eingebracht wird um dort die erforderlichen Reinraumbedinungen zu realisieren.
Diese Luftanlage kann entweder ein Gebläse in Verbindung mit einem Filter umfassen, um Umgebungsluft in das Gehäuse zu blasen oder einen Anschluss an eine zentrale Luftaufbereitungsanlage aufweisen, um Luft aus der zentralen Luftaufbereitungsanlage ins Innrere des Gehäuses zu leiten. In beiden Fällen ist die Anbringung eines, ggf. weiteren, Filters an der Luftanlage vorteilhaft, um eine Reinigung der einzublasenden Luft sicherzustellen.

In einer besonders bevorzugten Ausführungsform umfasst die Luftanlage am Übergang zum Gehäuse Laminarströmungselemente, um ein laminares Einströmen der Luft in das Innere des Gehäuses zu erreichen.

In einer weiteren, bevorzugten Ausführungsform umfasst die Entladevorrichtung eine Übergabeebene zur Anlage an einer Stellfläche der Gefriertrockungsanlage und eine Ausschubeinheit. Dabei hat es sich als vorteilhaft erwiesen, die Übergabebene nach oben schwenkbar und/oder nach unten absenkbar auszubilden. Hierdurch kann der Verfahrweg der Vorrichtung zur Gefriertrocknungsanlage oder weg von der Gefriertrocknungsanlage verkürzt werden, denn die entsprechend hoch- oder heruntergeklappte Übergabeebene kann dann besser an der Gefriertrocknungsanlage vorbeigeführt werden.

In einer anderen, ebenfalls bevorzugten Ausführungsform umfasst die Transportvorrichtung ein erstes Abförderband, mit dem die Vials von der Übergabeebene auf einen Umlenkteller gebracht werden und ein zweites Abförderband, mit dem die Vials von dem Abförderband zur Verschließvorrichtung im Inneren des Gehäuses gebracht werden. Durch die Kombination von ersten Abförderband, Umlenkteller und zweitem Abförderband ist eine kompakte Bauweise möglich , so dass die Transportvorrichtung auf der Station platz findet. Auch ist es hierdurch möglich, die Transportvorrichtung innerhalb des Gehäuses anzuordnen.
Während des Betriebes der Verschließvorrichtung ist die gesamte Station an die jeweilige Gefriertrocknungsanlage herangefahren, wobei die Übergabeebene aus dem Gehäuse herausragt und bis an die Stellfläche der Gefriertrocknungsanlage heranreicht. Das dabei ein ganz oder teilweise offener Spalt zwischen dem Gehäuse und der Gefriertrocknungsanlage verbleibt ist akzeptabel, denn durch die über die Luftanlage in das Innere des Gehäuses eingeblasene Luft entsteht im Gehäuse ein leichter Überdruck, so dass über den Spalt Luft entweichen kann mit der Folge, dass über den Spalt keine Kontamination ins Innere des Gehäuses gelangt.

Im Ergebnis ist es möglich, im Gehäuse verbesserte Reinraumbedingungen zu erreichen.

In einer weiteren, bevorzugten Ausführungsform ist an einer der Gefriertrocknungsanlage zugewandten Seite des Gehäuses eine Dockvorrichtung vorgesehen, mittels der das Gehäuse, vorzugsweise formschlüssig, an der Gefriertrocknungsanlage angebracht werden kann, um ein unbeabsichtigtes Verrutschen der Station während des Entladevorganges zu verhindern. In einer vorteilhaften Weiterbildung umfasst die Dockvorrichtung außerdem eine an einer Stirnseite des Gehäuses vorgesehene, vorzugsweise aufblasbare, Dichtung, um den Spalt zwischen Gehäuse und Gefriertrocknungsanlage abzudichten.

Weitere Vorteile der erfindungsgemäßen Vorrichtung ergeben sich aus der beigefügten Zeichnung und den nachstehend beschriebenen Ausführungsformen. Ebenso können die vorstehend genannten und die noch weiter ausgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter. Es zeigen:
- Fig. 1: eine schematisch dargestellte Ausführungsform einer erfindungsgemäßen Vorrichtung in einer von der Gefriertrocknungsanlage beabstandeten Position und bereit zum parallelen Verschieben;
- Fig. 2: eine schematische Darstellung der Vorrichtung gemäß Fig. 1 im angedockten Zustand, während der Entladung der Vials aus der Gefriertrocknungsanlage;
- Fig. 3: eine schematische Darstellung der Vorrichtung gemäß Fig. 1 in perspektivischer Ansicht.

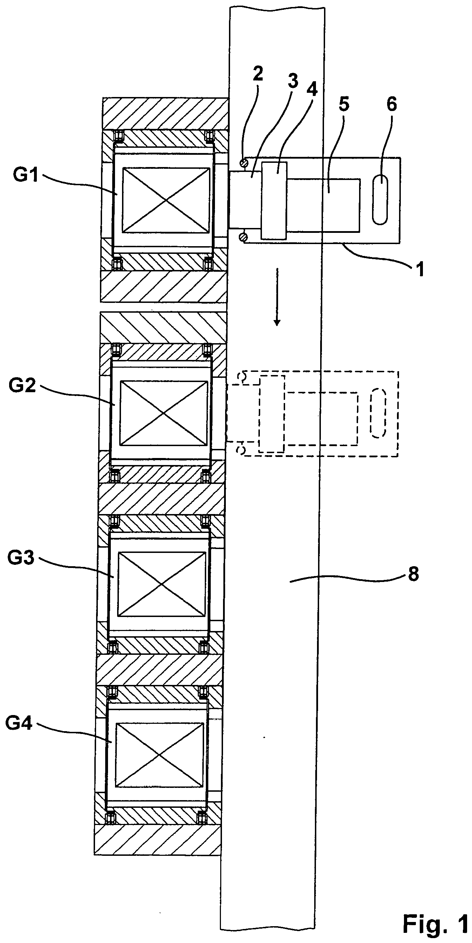

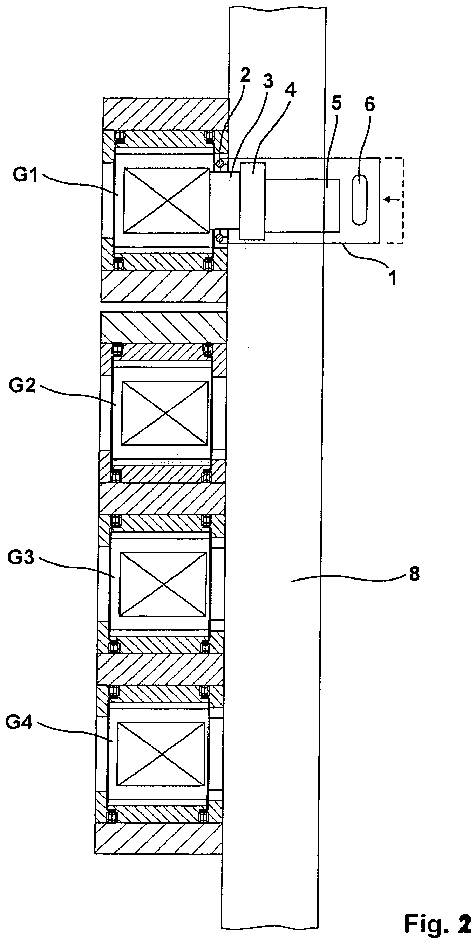

In den Figs. 1, 2 sind vier in Reihe aufgebaute Gefriertrocknungsanlagen G1 bis G4 dargestellt, wobei an einer ersten Gefriertrocknungsanlage G1 eine Vorrichtung zum pharmazeutischen Verschließen von mit einem hochreinen, vorzugsweise pharmazeutischen, Produkt gefüllten Vials im Anschluss an einen Gefriertrocknungsprozess angedockt ist.

Diese Vorrichtung umfasst eine in den Figuren 1 und 2 nicht erkennbare Station 7, auf der ein Gehäuse 1, eine Entladevorrichtung 3, eine Transportvorrichtung 4 und eine als Bördelmaschine 5 ausgeführte Verschließvorrichtung angeordnet sind. Im angedockten Zustand gemäß Fig. 2 sind die Bördelmaschine 5, die Transportvorrichtung 4 und teilweise auch die Entladevorrichtung 3 von dem auf der Station 7 ruhenden Gehäuse 1 abgedeckt, wobei an einer der Gefriertrocknungsanlage G1 zugewandten Seite des Gehäuses 1 eine Dockvorrichtung 2 vorgesehen ist, die die Station 7 sicher an der Gefriertrocknungsanlage G1 hält und den Spalt zwischen dem Gehäuse 1 und der Gefriertrocknungsanlage G1 mittels einer aufblasbaren Dichtung abdichtet.

Die Vorrichtung ist verfahrbar ausgebildet und kann sowohl parallel zu den Gefriertrocknungsanlagen G1 bis G4 entlang einer Führungsschiene 8 verfahren werden, als auch quer zu Führungsschiene 8, um an eine Gefriertrocknungsanlage G1 bis G4 herangeführt zu werden, beziehungsweise um dort abzudocken.

Am Gehäuse 1, vorzugsweise auf dessen Oberseite, ist eine Luftanlage 6 vorgesehen, mittels der Luft aus der Umgebung in das Innere des Gehäuses 1 gelangt. In einer anderen, hier nicht dargestellten Ausführungsform wird Luft aus einer zentralen Luftaufbereitungsanlage über die Luftanlage in das Innere des Gehäuses geleitet.

Dabei gelangt die Luft in laminarer Strömung an die Vials und an die Bördelmaschine 5 und verhindert, dass Verschmutzungen an oder in die Vials gelangen. Auch ein in der Luftanlage 6 integrierter Filter trägt zur Reinhaltung der Luft bei.

Das Gehäuse 1 besitzt am unteren Rand und/oder an seiner Unterseite hier nicht dargestelle Öffnungen, durch die die Luft entweichen kann. Hierdurch wird ein konstanter und auch laminarer Luftstrom im Inneren des Gehäuses 1 gewährleistet.

Im Ergebnis sind im Inneren des Gehäuses 1 verbesserte Reinraumbedingungen realisierbar.

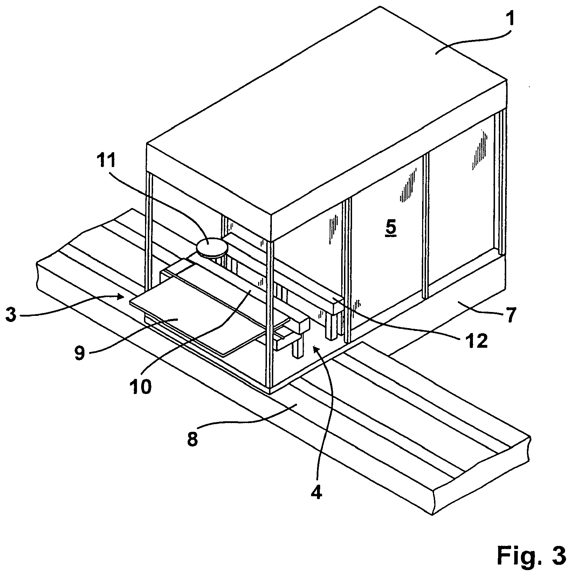

Die Entladevorrichtung 3 (siehe Fig. 3) umfasst eine Übergabeebene 9, die zum Verschieben der Vorrichtung quer zu den Gefriertrocknungsanlagen G1-G4 nach oben geschwenkt oder nach unten abgesenkt werden kann. An die Übergabeebene 9 schließt sich die Transportvorrichtung 4 mit einem ersten Abförderband 10 an, auf welchem die Vials mit an sich bekannten Entladeschiebern gebracht werden. Das Abförderband 10 bringt die Vials auf einen Umlenkteller 11, von wo die Vials auf ein zweites Abförderband 12 gelangen, welches die Vials zur Bördelmaschine 5 bringen, wo die Vials dann mit einem metallischen Verschluss pharmazeutisch verschlossen werden. Es versteht sich, dass auch ein nicht-metallischer Verschluss verwendet werden kann.

Nachfolgend ist der Ablauf einer Umsetzung der Vorrichtung detailiert beschrieben:
Zunächst wird die Übergabeebene 9 hochgeschwenkt, bevor die gesamte Vorrichtung mit der Station 7 entlang der linearen Führungsschiene 8 in die Position vor der Gefriertrocknungsanlage G1 gefahren wird (siehe Fig. 1). Danach wird die Vorrichtung quer zur Führungsschiene 8 auf die Gefriertrocknungsanlage G1 zu bewegt. In der Endposition angekommen wird das Gehäuse 1 sicher an der Gefriertrocknungsanlage G1 angedockt und der zwischen dem Gehäuse und der Gefriertrockungsanlage verbleibende Spalt wird durch die aufblasbare Dichtung 2 verschlossen.

Anschließend wird über die Luftanlage 6 eine laminare Strömung in das Innere des Gehäuses 1 eingebracht. Nun wird die Tür, hier eine als Schlitztür ausgebildete Schiebetür, der Gefriertrocknungsanlage G1 geöffnet und die Übergabeebene 9 bis an eine Stellfläche in der Gefriertrocknungsanlage G1 heruntergeschwenkt. Während dieser gesamten Zeit, und auch während des nachfolgenden Entladevorganges und während des Verschließens der Vials, herrschen, aufgrund der laminaren Strömung, im Inneren des Gehäuses verbesserte Reinraumbedingungen vorzugsweise der Klasse A. Der Innenraum der Gefriertrocknungsanlage G1 ist aufgrund des vorangehenden Gefriertrocknungsvorganges ohnehin aseptisch, so dass von der Gefriertrocknungsanlage keine Kontamination des in den Vials befindlichen Produktes zu befürchten ist.

Die Vials werden mit an sich bekannten und hier nicht näher dargestellten Entladeschiebern von den Stellflächen über die Übergabeebene 9 auf das Abförderband 10 gebracht und über den Umlenkteller 11 und das zweite Abförderband 12 zur Bördelmaschine 5 gebracht, wo sie mit einem metallischen Verschluß versehen und damit pharmazeutisch verschlossen werden. Über eine hier nicht näher dargestellte Schleuse werden die fertig verschlossenen Vials dann weitertransportiert.

## Patentansprüche

1. Vorrichtung zum pharmazeutischen Verschließen von mit einem hochreinen Produkt gefüllten Vials im Anschluss an einen Gefriertrocknungsprozess, insbesondere unter Reinraumbedingungen,
mit einer Station (7), auf der eine Verschließvorrichtung (5) zum pharmazeutischen Verschließen der Vials, ein die Verschließvorrichtung (5) abdeckendes Gehäuse (1), eine Entladevorrichtung (3) und eine Transportvorrichtung (4) zum Transportieren der Vials von einer Gefriertrocknungsanlage (G1-G4) zu der Verschließvorrichtung (5) untergebracht sind und mit einer am Gehäuse (1) angebrachten Luftanlage (6) zum Bereitstellen von hochreiner Luft innerhalb des Gehäuses (1), so dass im Inneren des Gehäuses (1) Reinraumbedingungen realisierbar sind,
**dadurch gekennzeichnet,**
**dass** die Station (7) verfahrbar ausgeführt ist, um die Vorrichtung von einer ersten Gefriertrocknungsanlage (G1-G4) zu einer zweiten Gefriertrocknungsanlage (G1-G4) zu bringen.

2. Vorrichtung nach Anspruch 1,
**dadurch gekennzeichnet,**
**dass** die Luftanlage (6) ein Gebläse aufweist, um Umgebungsluft in das Innere des Gehäuses (1) zu blasen und/oder dass die Luftanlage (6) an eine zentrales Luftgebläse angeschlossen ist, vorzugsweise wobei die Luftanlage (6) einen eigenen Filter aufweist.

3. Vorrichtung nach wenigstens einem der vorangehenden Ansprüche, **dadurch gekennzeichnet,**
**dass** an der Luftanlage (6) Laminarströmungselemente vorgesehen sind, damit die einströmende Luft laminar im Gehäuse (1) verströmt.

4. Vorrichtung nach wenigstens einem der vorangehenden Ansprüche, **dadurch gekennzeichnet,**
**dass** die Station (7) linear verfahrbar ausgeführt ist.

5. Vorrichtung nach wenigstens einem der vorangehenden Ansprüche, **gekennzeichnet durch**
eine Führungsschiene (8), entlang derer die Station (7) samt deren Aufbauten von einer ersten Gefriertrocknungsanlage (G1-G4) zu einer zweiten Gefriertrocknungsanlage (G1-G4) verfahrbar ist, insbesondere wobei die Führungsschiene (8) linear ausgebildet ist.

6. Vorrichtung nach wenigstens einem der vorangehenden Ansprüche, **dadurch gekennzeichnet,**
**dass** die Entladevorrichtung (3) eine Ausschubeinheit aufweist.

7. Vorrichtung nach wenigstens einem der vorangehenden Ansprüche **dadurch gekennzeichnet,**
**dass** die Entladevorrichtung (3) eine Übergabeebene (9) zur Anlage an einer Stellfläche der Gerfriertrocknungsanlage (G1-G4) aufweist, insbesondere wobei die Übergabeebene (9) schwenkbar und/oder absenkbar gehalten ist.

8. Vorrichtung nach wenigstens einem der vorangehenden Ansprüche, **dadurch gekennzeichnet,**
**dass** die Transportvorrichtung (4) ein erstes Abförderband (10) und ein zweites Abförderband (12) umfasst, wobei dazwischen ein Umlenkteller (11) vorgesehen ist.

9. Vorrichtung nach wenigstens einem der vorangehenden Ansprüche, **dadurch gekennzeichnet,**
**dass** am Gehäuse (1) eine Dockvorrichtung (2) zum Andocken des Gehäuses (1) an eine Öffnung der Gefriertrocknungsanlage (G1-G4) vorgesehen ist, insbesondere dass die Dockvorrichtung (2) eine aufblasbare Dichtung umfasst.

10. Vorrichtung nach einem der vorangehenden Ansprüche,
**dadurch gekennzeichnet,**
**dass** die Verschließvorrichtung (5) als Bördelmaschine ausgeführt ist.

## Claims

1. A device for pharmaceutical sealing of vials filled with a high-purity product following a freeze-drying process, in particular in clean room conditions, with a station (7), on which a sealing device (5) for pharmaceutical sealing of the vials, a housing (1) covering the sealing device (5), an unloading device (3) and a transport device (4) for transporting the vials from a freeze-dryer (G1-G4) to the sealing device (5) are accommodated and with a ventilation system (6) mounted on the housing (1) for providing high-purity air in the interior of the housing (1), so that clean room conditions can be provided in the interior of the housing (1),
**characterized in that**
the station (7) is designed to be displaceable, in order to bring the device from the first freeze dryer (G1-G4) to a second freeze dryer (G1-G4).

2. The device according to claim 1,
**characterized in that**
the ventilation system (6) comprises a blower in order to blow ambient air into the interior of the housing (1) and/or that the ventilation system (6) is connected to a central air blower, preferably wherein the ventilation system (6) has its own filter.

3. The device according to at least one of the afore-mentioned claims, **characterized in that**
laminary flow elements are provided at the air ventilation system (6) in order for the airflow flowing into the housing (1) to be laminary.

4. The device according to at least one of the afore-mentioned claims, **characterized in that**
the station (7) is designed to be linearly displaceable.

5. The device according to at least one of the afore-mentioned claims, **characterized by**
a guiding rail (8) along which the station (7) can be displaced together with its superstructure from a first freeze dryer (G1-G4) to a second freeze dryer (G1-G4), in particular wherein the guiding rail (8) is formed so that it is linear.

6. The device according to at least one of the afore-mentioned claims, **characterized in that**
the unloading device (3) comprises a discharge unit.

7. The device according to at least one of the afore-mentioned claims, **characterized in that**
the unloading device (3) comprises a transfer plane (9) to the installation at a storage surface of the freeze dryer (G1-G4), in particular wherein the transfer plane (9) is held so that it is pivotable and/or lowerable.

8. The device according to at least one of the afore-mentioned claims, **characterized in that**
the transport device (4) comprises a first discharge belt (10) and a second discharge belt (12), wherein a redirection plate (11) is provided in between.

9. The device according to at least one of the afore-mentioned claims, **characterized in that**
a docking device (2) is provided at the housing (1) for docking the housing (1) at an opening of the freeze dryer (G1-G4), in particular that the docking device (2) includes an inflatable seal.

10. The device according to one of the afore-mentioned claims, **characterized in that**
the sealing device (4) is designed as a crimping machine.

## Revendications

1. Dispositif de fermeture pharmaceutique de flacons contenant un produit de haute pureté à la suite d'un processus de lyophilisation, en particulier dans des conditions de salle propre, avec une station (7) abritant un dispositif de fermeture (5) pour la fermeture pharmaceutique des flacons, un boîtier (1) couvrant le dispositif de fermeture (5), un dispositif de déchargement (3) et un dispositif de transport (4) pour transporter les flacons d'une installation de lyophilisation (G1-G4) au dispositif de fermeture (5) et avec une installation de ventilation (6) fixée au boîtier (1) pour mettre à disposition de l'air de haute pureté à l'intérieur du boîtier (1), de manière à pouvoir réaliser des conditions de salle propre à l'intérieur du boîtier (1),
**caractérisé en ce que**
la station (7) est réalisée de manière à être déplaçable afin d'amener le dispositif d'une première installation de lyophilisation (G1-G4) à une seconde installation de lyophilisation (G1-G4).

2. Dispositif selon la revendication 1,
**caractérisé en ce que**
l'installation de ventilation (6) comporte un ventilateur pour souffler de l'air ambiant dans l'intérieur du boîtier (1) et/ou que l'installation de ventilation (6) est connectée à un ventilateur d'air central, de préférence où l'installation de ventilation (6) comporte son propre filtre.

3. Dispositif selon au moins une des revendications précédentes, **caractérisé en ce que**
des éléments d'écoulement laminaire sont prévus au niveau de l'installation de ventilation (6) pour que l'air pénétrant dans le boîtier (1) s'écoule de manière laminaire.

4. Dispositif selon au moins une des revendications précédentes, **caractérisé en ce que**
la station (7) est réalisée de manière à être déplaçable linéairement.

5. Dispositif selon au moins une des revendications précédentes, **caractérisé par**
un rail de guidage (8) le long duquel la station (7) et ses superstructures est déplaçable d'une première installation de lyophilisation (G1-G4) à une seconde installation de lyophilisation (G1-G4), en particulier où le rail de guidage (8) est réalisé de manière à ce qu'il soit linéaire.

6. Dispositif selon au moins une des revendications précédentes, **caractérisé en ce que**
le dispositif de déchargement (3) comporte une unité d'expulsion.

7. Dispositif selon au moins une des revendications précédentes, **caractérisé en ce que**
le dispositif de déchargement (3) comporte un plan de transfert (9) vers l'installation au niveau d'une surface de pose de l'installation de lyophilisation (G1-G4), en particulier où le plan de transfert (9) est maintenu de manière pivotable et/ou de manière à pouvoir être abaissé.

8. Dispositif selon au moins une des revendications précédentes, **caractérisé en ce que**
le dispositif de transport (4) comprend une première bande d'évacuation (10) et une seconde bande d'évacuation (12), où un plateau de déviation (11) est prévu entre elles.

9. Dispositif selon au moins une des revendications précédentes, **caractérisé en ce que**
un dispositif d'amarrage (2) est prévu au niveau du boîtier (1) pour amarrer le boîtier (1) à une ouverture de l'installation de lyophilisation (G1-G4), en particulier que le dispositif d'amarrage (2) comprend un joint gonflable.

10. Dispositif selon une des revendications précédentes, **caractérisé en ce que**
le dispositif de fermeture (5) est une machine de sertissage.
